# EUROPEAN PATENT SPECIFICATION

(11) **EP 0 853 625 B1**
(45) Date of publication and mention of the grant of the patent: **07.11.2001**
(21) Application number: 96928260.7
(22) Date of filing: 26.08.1996
(51) Int. Cl.: C07D 495/04

(54) **SYNTHESIS OF HYDROXYSULFONE AND RELATED COMPOUNDS**
SYNTHESE VON HYDROXYSULFONEN UND VERWANDTEN VERBINDUNGEN
SYNTHESE D'HYDROXYSULFONE ET DE COMPOSES APPARENTES

(30) Priority: 29.08.1995 US 2890 P; 13.02.1996 GB 9602852
(43) Date of publication of application: 22.07.1998
(73) Proprietor: MERCK & CO., INC., Rahway, New Jersey 07065 (US); Syngenta Limited, Haslemere, Surrey GU27 3JE (GB)
(72) Inventor: MATHRE, David, J., Rahway, NJ 07065 (US); SOHAR, Paul, Rahway, NJ 07065 (US); MOODY, David, London W1Y 6LN (GB); BLACKER, Andrew, J., London W1Y 6LN (GB)
(74) Representative: Hiscock, Ian James
(86) International application number: US9613634
(87) International publication number: WO9708173

(56) References cited:
- JOURNAL OF ORGANIC CHEMISTRY, vol. 56, no. 2, 1991, EASTON US, pages 763-769, XP002019742 T.K. JONES ET AL.: "An asymmetric synthesis of MK-0417. Observations on oxaazaborolidine-catalyzed reductions"
- JOURNAL OF ORGANIC CHEMISTRY, vol. 58, no. 10, 1993, EASTON US, pages 2880-2888, XP002019743 D.J. MATHERE: "A practical process for the preparation of tetrahydro-1-methyl-3,3-diphenyl-1H,3H-pyr rolo[1,2-c][1,3,2]oxazaborole-borane. A highly enantioselective stoichiometric and catalytic reducing agent"

## Description

### BACKGROUND OF THE INVENTION

The current therapy for control of elevated intraocular pressure (IOP) or ocular hypertension which is believed to be a factor in the onset and progress of glaucoma is typically effected with a variety of topically applied agents which fall within four categories: β-blockers, sympathomimetic agents, parasympatho-mimetic agents and cholinesterase inhibitors. The adjuvant oral administration of a carbonic anhydrase inhibitor (CAI) is practiced when the above-described topical agent's side effects limits its use and/or it fails to achieve adequate IOP control. The orally active CAI's can exhibit serious side-effects such as anorexia, gastrointestinal upset and parasthesias. Therefore an intense and ongoing search has been mounted for a topically active CAI that would not exhibit such side effects due to the route of administration and inherent target organ specificity. This search has resulted in the discovery of a class of compounds by Baldwin et al (US Patent 4,797,413) of general formula: 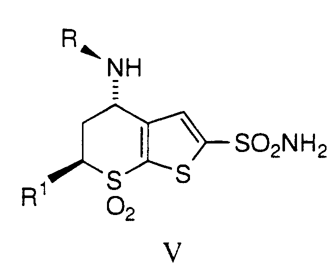 wherein R and R¹ are lower alkyl, especially dorzolamide, wherein R is ethyl and R¹ is methyl.

U.S. Patent 4,797,413 discloses a process for preparing the racemic modification of the alkyl 3-(thien-2-ylthio)butyrate and its homologs. The prior art process comprises addition of the 2-thienyl-thiol (II) across the double bond of a substituted acrylic acid (IV) to yield acid I: 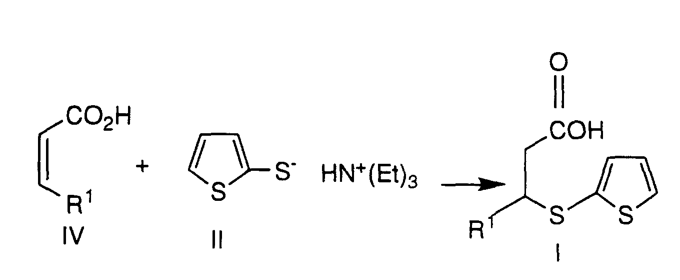 followed by synthesis of the final diastereomeric product, the isomers of which must be separated and each resolved to obtain the most active (S,S)-enantiomer. The isomer separations result in an automatic loss of the bulk of the chemical product.

U.S. Patent 4,968,815 discloses a process for preparing the acid of structural formula I: 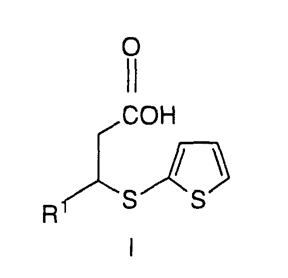 which comprises treating a nucleophile of structure II with a compound of structure III as shown: 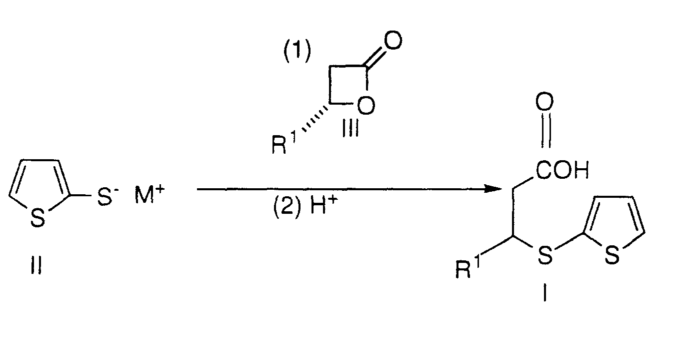 wherein the R groups are as hereinafter defined. U.S. Patent No. 4,968,814 and Blacklock et al., J. Org. Chem., 1993, 58, 1672-1679 also teaches a process for preparing the chiral intermediate formula I. However, these prior art processes involve many steps, employ heavy metal oxidants and are expensive and very time consuming.

It is therefore an object of this invention to provide a process for the synthesis of a hydroxysulfone which is more economical than previously possible and eliminates the use of heavy metal oxidants.

### SUMMARY OF THE INVENTION

This invention is concerned with an improved process for the synthesis of a hydroxysulfone of structural formula IV 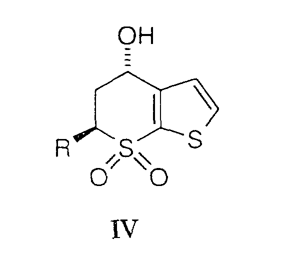 wherein R is hydrogen, C₁₋₄ alkyl, or C₁₋₄ alkoxy-C₁₋₄alkyl. The hydroxysulfone is a key intermediate in the synthesis of the compound of formula V: 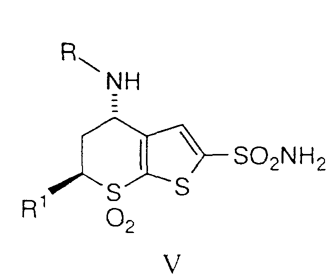 wherein R and R¹ are lower alkyl, especially dorzolamide, wherein R is ethyl and R¹ is methyl, a carbonic anhydrase inhibitor topically effective in the treatment of ocular hypertension and glaucoma.

The synthesis involves the preparation of a compound of structural formula: 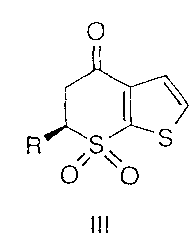 wherein R is hydrogen, C₁₋₄alkyl, or C₁₋₄alkoxy-C₁₋₄alkyl, comprising adding an anhydride belonging to the group consisting of trifluoroacetic anhydride, acetic anhydride, trichloroacetic anhydride, and mixtures thereof, to a solution containing a solvent and a compound of formula I: 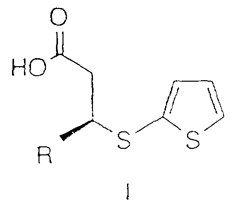 wherein R is defined as above, while maintaining a temperature of about -5 to about 50°C to produce a solution containing a compound of structural formula II: 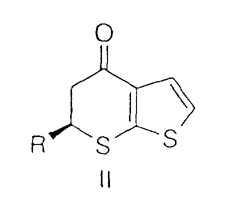 wherein R is defined as above, and adding to the said solution an oxidizing agent selected from hydrogen peroxide, t-butyl hydroperoxide, periodate, perchlorate and electrochemical oxidation, while maintaining a temperature of about 15°C to 80°C, to produce compound III;
characterized in that when said oxidizing agent is added to said solution, carhoxylic acid byproduct from the previous reaction is present therein.

The instant process reduces the reaction to a single batch process and eliminates the use of heavy metal oxidants, while retaining the high enantiomeric purity of the product.

### DETAILED DESCRIPTION OF THE INVENTION

The novel process of the present invention can be depicted as shown in Scheme I below:

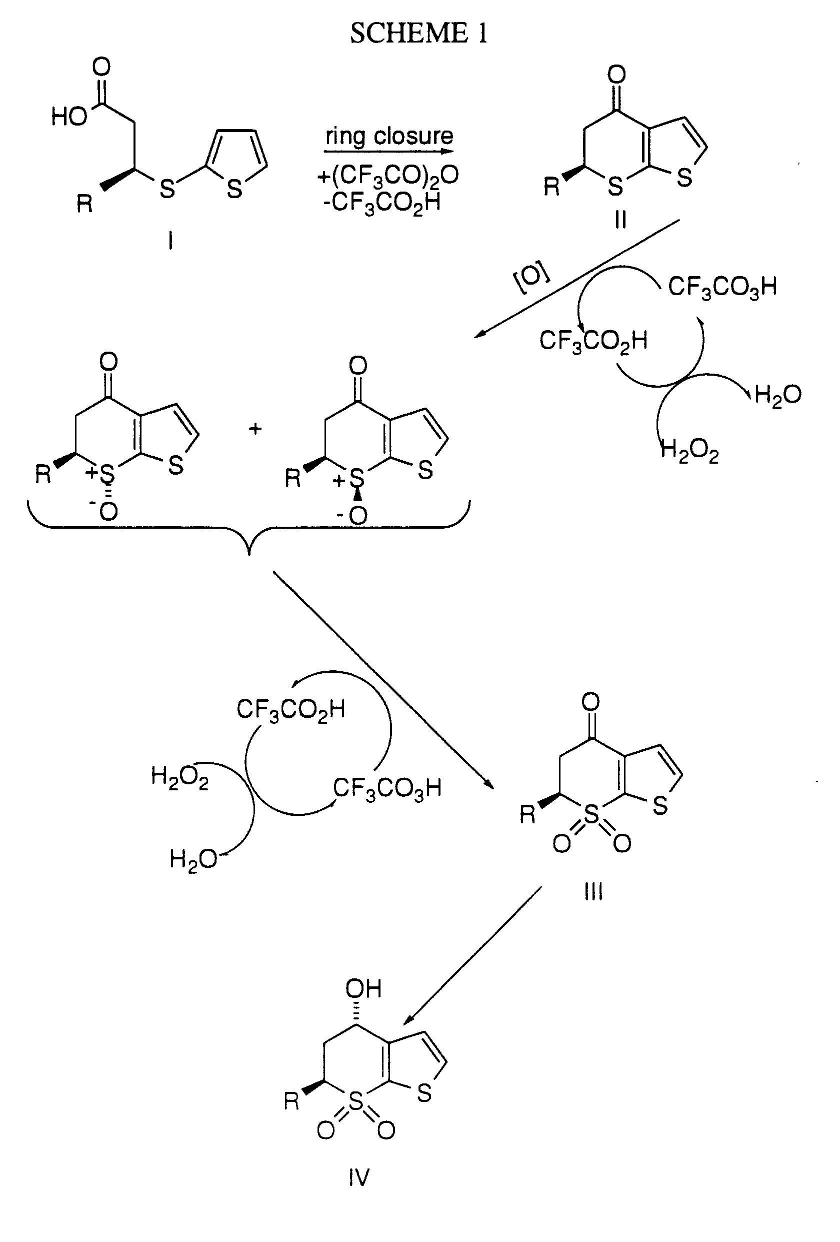

### Preparation of a Compound of structural formula IV:

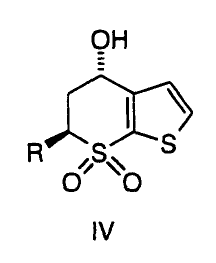

wherein R is hydrogen, C₁₋₄ alkyl, or C ₁₋₄ alkoxy-C ₁₋₄alkyl, comprises adding to a solution containing a solvent,belonging to a group consisting of toluene, benzene, cyclohexane, heptane, xylene, and the like, preferably toluene and a compound of formula I: 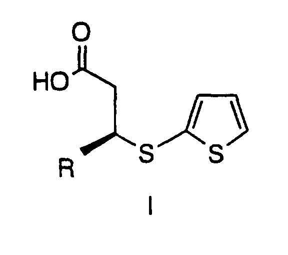 wherein R is described as above, an anhydride belonging to the group consisting of trifluoroacetic anhydride, acetic anhydride, trichloroacetic anhydride, and mixtures thereof, preferably trifluoroacetic anhydride, all optionally in the presence of acids such as carboxylic acids or inorganic acids, preferably phosphoric, polyphosphoric, orthophosphoric acids, or phosphorus pentoxide, while maintaining a temperature of about -5 to about 50°C, preferably from about 20°C to about 40°C, and most preferably from about 25°C to about 35°C, for about 1 to about eight hours to produce a solution containing a compound of structural formula II: 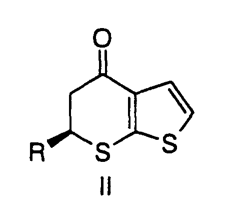 wherein R is described above. Any excess anhydride unused in the reaction is hydrolyzed by addition of a small amount of water, preferably from about 0.2 to about 2 equivalents of the original anhydride employed, prior to the addition of the oxidizing agent. To this solution is added at least about 2 mole equivalents and preferably from about 2 to about 4 mole equivalents of an oxidizing agent belonging to a group consisting of hydrogen peroxide, t-butyl hydroperoxide periodate, perchlorate electrochemical oxidation, preferably hydrogen peroxide, while maintaining a temperature of about 15°C to 80°C, preferably about 20°C to about 60°C, for about 1 to about 32 hours, preferably from about 2 to about 12 hours and most preferably from about 4 to about 8 hours, to produce Compound III, 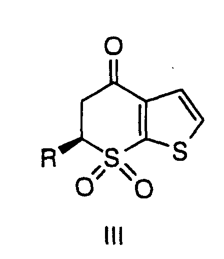 wherein R is described above, reducing Compound III to produce Compound IV and isolating compound IV.

The oxidation of the sulfide to the sulphoxide to the sulfone is performed directly after the ring closure by addition of the oxidizing agent (i.e., the cyclization and oxidation reaction are coupled in a single step). The reaction mixture at this point contains a byproduct from the previous reaction, the carboxylic acid. The oxidizing agent oxidizes the acid to the peracid, which then effects oxidation of the sulfide to the sulphoxide to the sulfone. A feature of this invention is that a reagent already present in the reaction mixture is converted to a suitable oxidizing agent and the need for a heavy metal oxidant is eliminated.

The reduction can be carried out by methods known to those skilled in the art. For example, Compound III can be reduced by subjecting it to the action of a microorganism such as Ambrosiozyma, Arthroascus Rhodotorula, Saccharomycopsis, Trichosporon and the like, which reduces the oxygen atom of carbonyl group at the 4-position of the thienothiopyran ring to the hydroxyl group. Chemical reducing agents can also be used. These include lithium aluminum hydride, diisobutyl aluminum hydride, aluminum hydride, lithium aluminum, tri-t-butoxy hydride, diborane, and the like.

The reaction can be quenched by addition of the reaction mixture or part of the reaction mixture, preferably the aqueous oxidizing phase of a biphasic solution to aqueous ethyl acetate, or aqueous sodium sulphite or bisulphite or addition of aqueous ethyl acetate to the reaction mixture, or aqueous sodium sulphite, or bisulphite. Ethyl acetate can be replaced by n-butyl acetate, methyl *t*-butyl ether methyl ethyl ketone, methyl isobutyl ketone, and the like. Hexane can be replaced by pentane, cyclohexane, cyclopentane, heptane, petroleum ether, and the like. Brine can be composed of aqueous solutions of sodium chloride, calcium chloride, sodium sulfate, calcium sulfate, magnesium sulfate, potassium carbonate, and the like.

The reaction steps are exemplified by the Example that follows. The product of the novel process of this invention is a topically effective carbonic anhydrase inhibitor useful in the treatment of ocular hypertension. It is administered topically to the eye usually as a solution, comprising about 0.1 % to 15% by weight of compound, one or two drops at a time, one to four times a day.

### EXAMPLE 1

Trifluoroacetic anhydride (106 mL, 0.75 mol) was added to a solution of (S)-3-(2-thienylthio)butyric acid (113.8 g, 0.56 mol) in toluene (750 mL), which had been cooled to -5°C. Addition was done at a rate such that the temperature of the reaction mixture remained at 0-5°C. The reaction was allowed to warm to 20-25°C. The reaction was monitored by HPLC, and the ring closure reaction was found to be complete after 2 hours. The reaction mixture was then cooled to 0°C, and H₂O₂ (30%, 233 mL) was slowly added so that the temperature of the reaction mixture remained at 25 to 30°C. Addition of H₂O₂ was highly exothermic. The temperature of the reaction mixture continued to rise for one hour after addition of peroxide, and the temperature was controlled by cooling the flask with an ice bath. The reaction was allowed to run for 24 hours at 20-25°C. The reaction mixture was then cooled to -5°C, and a saturated solution of sodium bisulfite (1500 mL H₂O, 180 g Na₂SO₃) was slowly added so as to keep the temperature below 5°C. The reaction mixture was allowed to warm to 25°C, the layers were separated, and the aqueous layer washed with toluene (500 mL). The combined organic layers were then washed with water (500 mL), and concentrated to a volume of 170 mL. Hexane (550 mL) was added, and the solution cooled to 0°C. The compound was isolated by filtration to produce 92.8 g (80% yield from the acid) of crude product. The compound was recrystallized by dissolving in isopropyl alcohol (232 mL) and heating to 75-80°C. H₂O (367 mL) was then added at a rate so as to maintain the temperature at 60-65°C. The solution was allowed to cool to room temperature, and the compound crystallized. The solution was further cooled to 0°C, and the product was isolated by filtration. The product was washed with H20 which had been cooled to 0°C (2 x 60 mL), and 84.8 g (91.5% yield for the recrystallization) of ketosulfone was isolated.

Analysis: ¹H NMR (CDCl₃) δ7.65 (d, 1 H, *J*=5.1 Hz), 7.48 (d, 1 H, *J*=5.1 Hz), 3.92 (m, 1 H), 3.22 (d, 2 H, *J*=2.5 Hz), 1.56 (d, 3 H, *J*=6.9 Hz); ¹³C NMR (CDCl₃) δ 187.1, 147.2, 140.4, 131.3, 126.6, 58.4, 45.1, 12.2.

### EXAMPLE 2

| | | | | | | |
|---|---|---|---|---|---|---|
| Scale: 0.1 gram mole to make 12.8 grams of FC4010 Stage 5 sulphone at approximately 98% strength in 58% isolated yield | | | | | | |

| Materials | Hazard catagory | MWt | Actual Wt(g) | 100% Wt(g) | Gram Moles | Molar Ratio |
|---|---|---|---|---|---|---|
| FC4010 Stage 3 acid20% soln. in tol. | H2 | 202 | 101 | 20.2 | 0.1 | 1.0 |
| | | | | | | |
| Trifluoroacetic anhydride 99% | H2 | 210 | 25.4 | 25.2 | 0.12 | 1.2 |
| | | | | | | |
| 30%w/w Hydrogen peroxide | H2 | 34 | 23.8 | 7.14 | 0.21 | 2.1 |
| | | | | | | |
| 20%w/w Sodium bisulphite | H2 | 104 | 52.0 | 10.4 | 0.1 | 1.0 |
| | | | | | | |
| Isopropanol | M | 60 | 30.0 | 30.0 | 0.5 | 5.0 |

A 250 ml RBQF flask fitted with PTFE paddle stirrer, short Dean & Stark (D&S) side arm leading to a double surface condenser fitted with N2 bubbler, thermometer and 50 ml dropping funnel is employed in the reaction. A dry reaction flask is purged with nitrogen and charged with FC4010 Stage 3 acid solution (101 g) and the pH checked to ensure that it is acidic (pH 4). The D&S side arm is filled with dry toluene. Vacuum (about 80-100 mm Hg) is applied and the flask contents heated to reflux and azeotropically dried. The toluene solution is cooled to 35°C and the vacuum released with nitrogen. A slow nitrogen bleed is maintained throughout the preparation.

Trifluoroacetic anhydride (25.4 g) is charged to the dropping funnel and added dropwise to the flask contents over a period of 90 minutes whilst maintaining the temperature at 30-35°C. The solution is held at 30-35°C for a further 1.0 to 1.5 hours by which time cyclization is judged to be complete by GC analysis.

The temperature is raised to 40-45°C (agitator speed 300 rpm) and water (1.8 g) added dropwise over 10 minutes. An exotherm occurs as the excess trifluoroacetic anhydride is hydrolyzed. 30% w/w hydrogen peroxide solution (23.8 g) is charged to the dropping funnel and added dropwise to the flask over a period of 5 hours whilst maintaining the temperature at 45-50°C by applying cooling as necessary (a bath temperature of 35-40°C is adequate to maintain the temperature). On completion of the addition the reaction mixture (now a two phase system) is stirred for a further 1.0 hour to complete the oxidation (GC shows the required product at RT 10.92 with the intermediate sulphoxide at RT 10.82).

The flask contents are cooled to ambient temperature and drowned into 20% sodium bisulphite solution (52 g) contained in a 500 ml stirred flask, whilst maintaining the temperature below 25°C. After stirring for 10 minutes the mixture is transferred to a separating funnel and the lower aqueous layer separated off. The aqueous phase is extracted with toluene (25 ml) and the combined toluene phases washed with 0.5% sodium bisulphite solution (1 x 50 ml) followed by water (2 x 50 ml). The pH of the washes are checked to ensure that the pH of the final wash is between pH4-5. More water washes are applied if necessary. The combined State 4/5 reaction yield is about 74%.

The toluene solution is charged to a dry flask and toluene distilled off under vacuum (50 mmHg) until a total of 70 g have been collected. The vacuum is released with nitrogen and the flask contents cooled to 50°C. Isopropanol (30 g) is added rapidly and the resulting solution cooled to 20°C over 1.0 hour and then held at this temperature for 2-4 hours until crystallization is complete. The crystalline product is filtered off, washed with 15-20°C isopropanol (2 x 10 ml) and finally dried in a vacuum oven at 30-35°C. The weight of the product was 12.78 grams (Strength - GC vs. Int. Std.=98%) and recovery from crude product was 78%.

## Claims

1. Preparation of a Compound of structural formula: 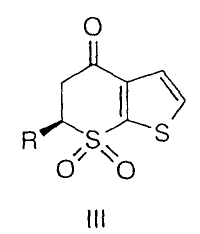 wherein R is hydrogen, C₁₋₄alkyl, or C₁₋₄alkoxy-C₁₋₄alkyl, comprising adding an anhydride belonging to the group consisting of trifluoroacetic anhydride, acetic anhydride, trichloroacetic anhydride, and mixtures thereof, to a solution containing a solvent and a compound of formula I: 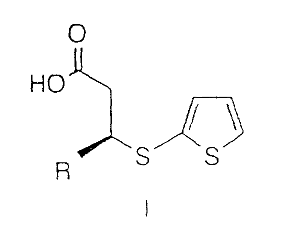 wherein R is defined as above, while maintaining a temperature of about -5 to about 50°C to produce a solution containing a compound of structural formula II: 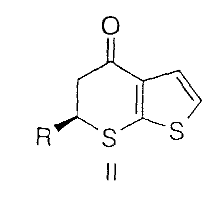 wherein R is defined as above, and adding to the said solution an oxidizing agent selected from hydrogen peroxide, t-butyl hydroperoxide, periodate, perchlorate and electrochemical oxidation, while maintaining a temperature of about 15°C to 80°C, to produce compound III; **characterized in that** when said oxidizing agent is added to said solution, carboxylic acid byproduct from the previous reaction is present therein.

2. The process of claim 1 wherein the anhydride is added in the presence of an acid.

3. The process of claim 1 wherein excess and unused anhydride is hydrolyzed by addition of about 0.2 to about 2 equivalents of the original amount of anhydride of water prior to the addition of the oxidizing agent.

4. The process of claim 1 wherein R is methyl, the anhydride belongs to a group consisting of trifluoroacetic anhydride. acetic anhydride, or trichloroacetic anhydride and the solvent belongs to a group consisting of toluene, benzene, cyclohexane, heptane and xylene.

5. The process of claim 4 wherein the anhydride is trifluoroacetic anhydride, the solvent is toluene and the oxidizing agent is hydrogen peroxide.

6. The process of claim 1 wherein a temperature of about 20°C to 40°C is maintained while the anhydride is added and the oxidizing temperature is 20°C to about 60°C.

7. The process of claim 2 wherein the acid belongs to the group consisting of carboxylic acids and inorganic acids.

8. The process of claim 7 wherein the inorganic acids belong to the group consisting of phosphoric, polyphosphoric, orthophosphoric acids and phosphorus pentoxide.

9. The process of claim 5 wherein at least 2 mole equivalents of oxidising agent are added.

10. A process for the preparation of a Compound IV of structural formula: 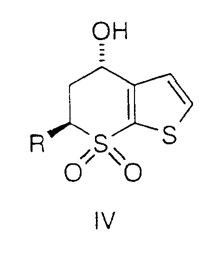 wherein R is as defined in claim 1, comprising:
preparing a compound III of formula: 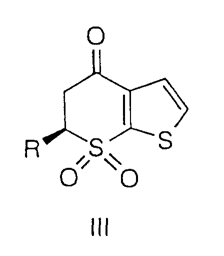 wherein R is as defined in claim 1, by a process according to any of claims 1-9, reducing Compound III to produce Compound IV and isolating Compound IV.

## Patentansprüche

1. Herstellung einer Verbindung der Strukturformel: 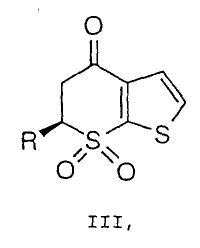 worin R Wasserstoff, C₁₋₄-Alkyl oder C₁₋₄-Alkoxy-C₁₋₄-alkyl ist, umfassend die Zugabe eines Anhydrids, das zur Gruppe, bestehend aus Trifluoressigsäureanhydrid, Essigsäureanhydrid, Trichloressigsäureanhydrid und Mischungen davon, gehört, zu einer Lösung, die ein Lösungsmittel und eine Verbindung der Formel I: 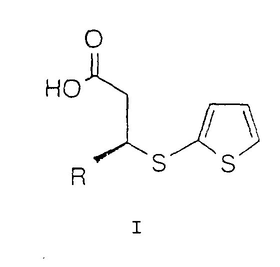 enthält, worin R wie oben definiert ist, während eine Temperatur von etwa -5°C bis etwa 50°C aufrechterhalten wird, um eine Lösung zu erzeugen, die eine Verbindung der Strukturformel II: 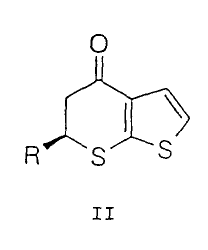 enthält, worin R wie oben definiert ist, und die Zugabe eines Oxidationsmittels, ausgewählt aus Wasserstoffperoxid, t-Butylhydroperoxid, Periodat, Perchlorat und elektrochemischer Oxidation, zu der Lösung, während eine Temperatur von etwa 15°C bis 80°C aufrechterhalten wird, um Verbindung III zu erzeugen,
**dadurch gekennzeichnet, daß**, wenn das Oxidationsmittel zu der Lösung hinzugegeben wird, sich darin Carbonsäure-Nebenprodukt aus der vorhergehenden Reaktion befindet.

2. Das Verfahren nach Anspruch 1, bei dem das Anhydrid in Gegenwart einer Säure zugegeben wird.

3. Das Verfahren nach Anspruch 1, bei dem überschüssiges und unverbrauchtes Anhydrid durch Zugabe von etwa 0,2 bis etwa 2 Äquivalenten Wasser, bezogen auf die ursprüngliche Menge Anhydrid, vor der Zugabe des Oxidationsmittels hydrolysiert wird.

4. Das Verfahren nach Anspruch 1, bei dem R Methyl ist, das Anhydrid zu einer Gruppe, bestehend aus Trifluoressigsäureanhydrid, Essigsäureanhydrid oder Trichloressigsäureanhydrid, gehört und das Lösungsmittel zu einer Gruppe, bestehend aus Toluol, Benzol, Cyclohexan, Heptan und Xylol, gehört.

5. Das Verfahren nach Anspruch 4, bei dem das Anhydrid Trifluoressigsäureanhydrid ist, das Lösungsmittel Toluol ist und das Oxidationsmittel Wasserstoffperoxid ist.

6. Das Verfahren nach Anspruch 1, bei dem während der Zugabe des Anhydrids eine Temperatur von etwa 20°C bis 40°C aufrechterhalten wird und die Oxidationstemperatur 20°C bis etwa 60°C beträgt.

7. Das Verfahren nach Anspruch 2, bei dem die Säure zur Gruppe, bestehend aus Carbonsäuren und anorganischen Säuren, gehört.

8. Das Verfahren nach Anspruch 7, bei dem die anorganischen Säuren zur Gruppe, bestehend aus Phosphor-, Polyphosphor-, Orthophosphorsäuren und Phosphorpentoxid, gehören.

9. Das Verfahren nach Anspruch 5, bei dem wenigstens 2 Moläquivalente Oxidationsmittel zugegeben werden.

10. Ein Verfahren zur Herstellung einer Verbindung IV der Strukturformel: 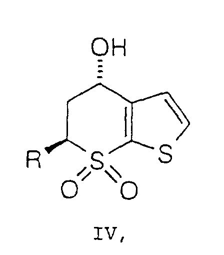 worin R wie in Anspruch 1 definiert ist, umfassend die Herstellung einer Verbindung III der Formel: 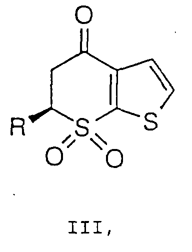 worin R wie in Anspruch 1 definiert ist, durch ein Verfahren gemäß irgendeinem der Ansprüche 1-9, die Reduktion von Verbindung III, um Verbindung IV zu erzeugen, und die Isolierung von Verbindung IV.

## Revendications

1. Préparation d'un composé de formule développée: 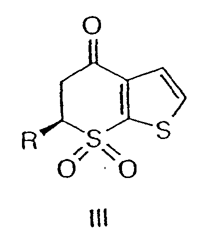 dans laquelle R est un atome d'hydrogène ou un groupe alkyle en C₁₋₄ ou alcoxy(en C₁₋₄)-alkyle(en C₁₋₄), comprenant l'addition d'un anhydride appartenant au groupe constitué par l'anhydride trifluoroacétique, l'anhydride acétique, l'anhydride trichloroacétique, et leurs mélanges, à une solution contenant un solvant et un composé de formule I 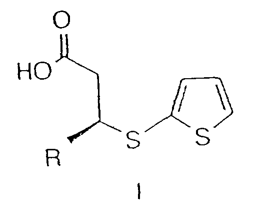 dans laquelle R est tel que défini ci-dessus, tout en maintenant une température d'environ -5 à environ 50°C pour produire une solution contenant un composé de formule développée II: 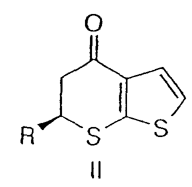 dans laquelle R est tel que défini ci-dessus, et l'addition à ladite solution d'un agent oxydant choisi parmi le peroxyde d'hydrogène, l'hydroperoxyde de t-butyle, le periodate, le perchlorate, et une oxydation électrochimique, tout en maintenant une température d'environ 15°C à 80°C, pour produire le composé III;
**caractérisée en ce que**, quand ledit agent oxydant est ajouté à ladite solution, on est en présence de sous-produit acide carboxylique de la réaction précédente.

2. Procédé selon la revendication 1 dans lequel l'anhydride est ajouté en présence d'un acide.

3. Procédé selon la revendication 1 dans lequel l'anhydride en excès et non utilisé est hydrolysé par addition d'environ 0,2 à environ 2 équivalents de la quantité initiale d'anhydride et d'eau avant l'addition de l'agent oxydant.

4. Procédé selon la revendication 1 dans lequel R est un groupe méthyle, l'anhydride appartient à un groupe constitué par l'anhydride trifluoroacétique, l'anhydride acétique ou l'anhydride trichloroacétique, et le solvant appartient à un groupe constitué par le toluène, le benzène, le cyclohexane, l'heptane et le xylène.

5. Procédé selon la revendication 4 dans lequel l'anhydride est l'anhydride trifluoroacétique, le solvant est le toluène et l'agent oxydant est le peroxyde d'hydrogène.

6. Procédé selon la revendication 1 dans lequel une température d'environ 20°C à 40°C est maintenue pendant l'addition de l'anhydride et la température d'oxydation est de 20°C à environ 60°C.

7. Procédé selon la revendication 2 dans lequel l'acide appartient au groupe constitué par les acides carboxyliques et les acides inorganiques.

8. Procédé selon la revendication 7 dans lequel les acides inorganiques appartiennent au groupe constitué par les acides phosphorique, polyphosphorique, orthophosphorique, et le pentoxyde de phosphore.

9. Procédé selon la revendication 5 dans lequel au moins 2 équivalents molaires d'agent oxydant sont ajoutés.

10. Procédé de préparation d'un composé IV de formule développée: 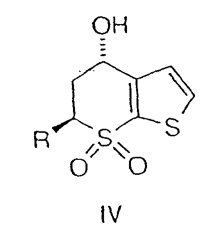 dans laquelle R est tel que défini dans la revendication 1, comprenant: la préparation d'un composé III de formule: 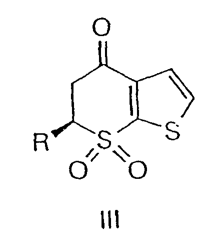 dans laquelle R est tel que défini dans la revendication 1, par un procédé selon l'une quelconque des revendications 1-9, la réduction du composé III pour produire le composé IV et l'isolement du composé IV.
